# EUROPEAN PATENT APPLICATION

(11) **EP 2 856 927 A1**
(43) Date of publication of application: **08.04.2015**
(21) Application number: 13797909.2
(22) Date of filing: 30.04.2013
(51) Int. Cl.: A61B 1/04, A61B 1/00, G02B 23/24

(54) **STEREOSCOPIC ENDOSCOPE SYSTEM**

(30) Priority: 29.05.2012 JP 2012122283
(71) Applicant: Olympus Corporation, Shibuya-ku Tokyo 151-0072 (JP)
(72) Inventor: IKEDA, Hiromu, Tokyo 151-0072 (JP)
(74) Representative: Schicker, Silvia
(86) International application number: PCT/JP2013/062670
(87) International publication number: WO 2013/179855

(57) **Abstract**

To simultaneously observe not only a principal part but also a surrounding wide range, the present invention provides a stereoscopic endoscope system (1) including an endoscope (2) including a plurality of image capturing units (22) which photograph a subject to acquire images at a leading edge of a long narrow insertion unit (21) insertable into the subject, an image processing apparatus (3) that processes the images obtained by the image capturing units (22) in the endoscope 2 to generate a display image, and an image display apparatus (4) that displays the display image generated by the image processing in the image processing apparatus (3), in which the image processing apparatus (3) generates the display image having a three-dimensional image generated from the plurality of images acquired by the plurality of image capturing units (22) arranged in a central part of the display image and having a two-dimensional image arranged around the outer periphery of the three-dimensional image.

## Description

### {Technical Field}

The present invention relates to a stereoscopic endoscope system used in a surgical operation or the like.

### {Background Art}

In recent years, an endoscope has frequently been used in a surgical operation. Further, in such an endoscope, a stereoscopic endoscope system has been used to perform magnifying observation of a minute surgical site. In an operation of an organ composed of a minute tissue, not only observation of significantly important and delicate tissues such as a blood vessel and a nerve serving as targets but also a treatment for actually connecting the blood vessel and the nerve and removing a tumor while avoiding the blood vessel and the nerve may be performed in a significant narrow region. Thus, in the stereoscopic endoscope system, to not only perform magnifying observation of an observation target but also stereoscopically grasp the observation target to perform the treatment becomes an important function.

Generally, a person uses various types of information as cues when stereoscopically grasping an object with his/her eyes. The information includes a binocular parallax, a sense of perspective (a far object looks small and a near object looks large), a blur in a depth direction, an overlap of objects, a past experience, a knowledge, and a memory. Suppose a case where an unknown sample (a never-seen-before object) is placed on a uniform stand, and is observed not to be shaded by being illuminated from all directions. In such a case, the overlap of objects, the knowledge, and the past experience among the aforementioned information for stereoscopically grasping the object become useless, and the binocular parallax becomes effective as a clue to know the depth of the object. A medical stereoscopic image capturing apparatus performs stereoscopic viewing using the binocular parallax, to obtain depth information about the observation target.

In performing the operation, not only a site to be observed but also the periphery thereof preferably enters a visual field. When a specific treatment is performed, for example, if any effect appears in its surrounding portion, the effect can be visually recognized. Thus, a viewing angle (an angle of view) of an observation image to be obtained is preferably wide.

However, when an image for a left eye and an image for a right eye, which have a parallax therebetween, overlap each other, if the angle of view is wide, the image is greatly distorted around the outer periphery of an obtained image so that information becomes difficult to grasp.

On the other hand, PTL 1 enables display by switching between a three-dimensional mode in which stereoscopic display is performed and a wide-angle two-dimensional mode in which a wide range can be observed.

PTL 2 discloses a configuration in which a mode in which stereoscopic display is performed using an image for a left eye and an image for a right eye and a mode in which an image observable in a wider range than that in the stereoscopic display is displayed can be switched.

### {Citation List}

### {Patent Literature}

{PTL 1} Japanese Unexamined Patent Application, Publication No. Hei 9-5643
{PTL 2} Japanese Unexamined Patent Application, Publication No. 2004-320722

### {Summary of Invention}

### {Technical Problem}

However, in methods described in PTL 1 and PTL 2, a stereoscopic display image and an image for displaying a wide range cannot simultaneously be observed, and need to be alternately switched. Therefore, when a wide range surrounding a principal part is desired to be observed while a stereoscopic display image is observed for the principal part, the images need to be switched, and thus there is room for improvement in usability.

The present invention has been made in view of such circumstances, and is directed to providing a stereoscopic endoscope system capable of simultaneously and reliably observing not only a principal part but also a surrounding wide range.

### {Solution to Problem}

To solve the aforementioned problem, a stereoscopic endoscope system according to the present invention adopts the following solutions:
That is, an aspect of the present invention is to provide a stereoscopic endoscope system including an endoscope including a plurality of image capturing units which photograph a subject to acquire images, at a leading edge of a long narrow insertion unit insertable into the subject, an image processing apparatus that processes the images acquired by the image capturing units of the endoscope to generate a display image, and an image display apparatus that displays the display image generated by the image processing in the image processing apparatus, in which the image processing apparatus generates the display image having a three-dimensional image generated from the plurality of images acquired by the plurality of image capturing units arranged in a central part of the display image and having a two-dimensional image arranged around the outer periphery of the three-dimensional image.

Thus, a site to be treated can be observed without being distorted by the two-dimensional image arranged around the outer periphery of the three-dimensional image while being stereoscopically observed by the three-dimensional image arranged in the central part of the display image displayed by the image display apparatus.

In the aforementioned aspect, the image processing apparatus may generate the three-dimensional image from a first image acquired by a first image capturing unit and a second image acquired by a second image capturing unit and having a parallax from the first image, and obtain the two-dimensional image around the outer periphery of the three-dimensional image from the first image.

That is, the first image is displayed on the entire area of the display image, and the second image is displayed while overlapping the first image in a portion where the three-dimensional image is to be displayed. Thus, the display image having the three-dimensional image arranged in its central part and having the two-dimensional image arranged in its outer peripheral part can be generated.

In the aforementioned aspect, the first image capturing unit may have a larger angle of view than that of the second image capturing unit.

Thus, the first image by the first image capturing unit having the larger angle of view can be used as the three-dimensional image and the two-dimensional image around the outer periphery thereof, and the second image by the second image capturing unit having the smaller angle of view can be used as the three-dimensional image.

Not the angle of view but the lens diameter of the first image capturing unit may be made larger than the lens diameter of the second image capturing unit.

In the aforementioned aspect, the endoscope may perform photographing by arranging the first image capturing unit having the larger angle of view than that of the second image capturing unit on the side of the dominant eye of a user.

Thus, the user can visually recognize the three-dimensional image and the two-dimensional image around the outer periphery thereof without having an uncomfortable feeling.

In the aforementioned aspect, the first image capturing unit having the larger angle of view and the second image capturing unit having the smaller angle of view may have the same number of pixels.

Thus, in a region of the three-dimensional image generated using the first image by the first image capturing unit and the second image by the second image capturing unit, the number of pixels in the second image by the second image capturing unit can be made large. Therefore, the image quality of the three-dimensional image can be enhanced.

In the aforementioned aspect, the image processing apparatus may generate the three-dimensional image from the first image captured by the first image capturing unit and the second image captured by the second image capturing unit and having a parallax from the first image, generate a third image having an optical axis midway between the first image capturing unit and the second image capturing unit from the first image and the second image, and uses the third image as the two-dimensional image.

In the aforementioned aspect, the first image capturing unit may have a larger angle of view than that of the second image capturing unit, and the first image capturing unit and the second image capturing unit may have the same number of pixels.

In the aforementioned aspect, the image processing apparatus may set a luminance of a boundary portion between the three-dimensional image and the two-dimensional image lower than those of the three-dimensional image and the two-dimensional image. Thus, the boundary portion becomes inconspicuous.

In the aforementioned aspect, the image processing apparatus may generate a boundary display portion representing a boundary between the three-dimensional image and the two-dimensional image, to clearly specify the boundary portion between the three-dimensional image and the second dimensional image.

Further, in the aforementioned aspect, the image processing apparatus may generate the three-dimensional image from the first image captured by the first image capturing unit and the second image captured by the second image capturing unit and having a parallax from the first image while gradually reducing the parallax between the first image and the second image, which form the three-dimensional image, toward the two-dimensional image from the three-dimensional image in the boundary portion between the three-dimensional image and the two-dimensional image. Thus, the boundary portion between the three-dimensional image and the two-dimensional image can be made inconspicuous.

### {Advantageous Effects of Invention}

According to the present invention, the effect of simultaneously observing not only a principal part but also a surrounding wide range with high visibility is produced.

### {Brief Description of Drawings}

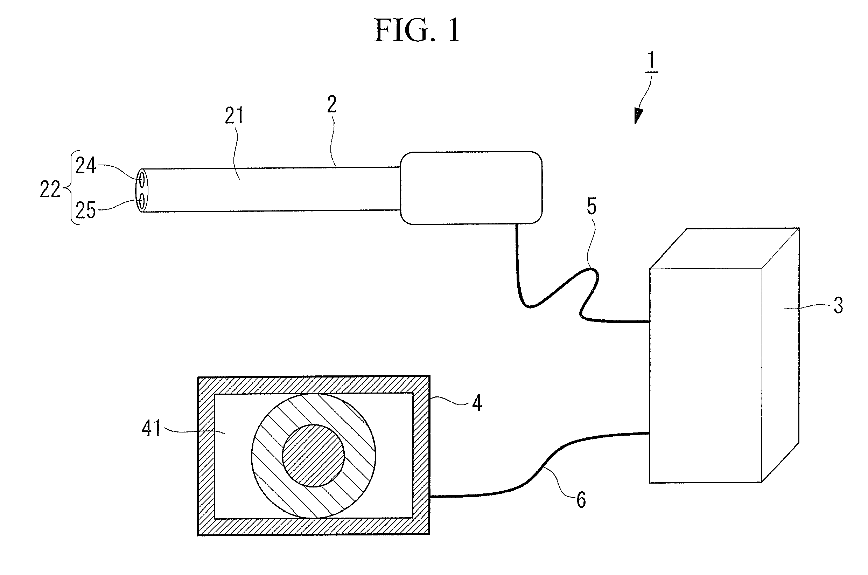

{Fig. 1}
   Fig. 1 is a schematic configuration diagram illustrating a stereoscopic endoscope system according to a first embodiment of the present invention.
{Fig. 2}
   Fig. 2 is a diagram illustrating a configuration of an image capturing unit in the first embodiment, where Fig. 2 (a) illustrates images respectively acquired by two photographing lenses, and Fig. 2 (b) illustrates an image acquired by a virtual lens based on the two photographing lenses.
{Fig. 3}
   Fig. 3 is a diagram illustrating an example of an image displayed in an image display unit in the first embodiment.
{Fig. 4}
   Fig. 4 is a diagram illustrating a configuration of an image capturing unit in a second embodiment, where Fig. 4 (a) illustrates a case where the dominant eye is a right eye, and Fig. 4 (b) illustrates a case where the dominant eye is a left eye.
{Fig. 5}
   Fig. 5 is a diagram illustrating an example of an image displayed in image display units in second and third embodiments.
{Fig. 6}
   Fig. 6 is a diagram illustrating a configuration of an image capturing unit in the third embodiment.
{Fig. 7}
   Fig. 7 is a diagram illustrating another example of an image displayed in the image display unit.
{Fig. 8}
   Fig. 8 is a diagram illustrating still another example of an image displayed in the image display unit.

### {Description of Embodiments}

A plurality of embodiments of a stereoscopic endoscope system 1 according to the present invention will be described below with reference to the drawings.

### {First Embodiment}

First, an overall configuration of the stereoscopic endoscope system 1, which is common among the plurality of embodiments of the present invention, will be described with reference to Fig. 1.

As illustrated in Fig. 1, the stereoscopic endoscope system 1 includes an endoscope 2 that is inserted into a body cavity (subject) of a person to be operated and captures an image of the inside of the body cavity, an image processing apparatus 3 that processes the image captured by the endoscope 2, and an image display apparatus 4 that displays the image processed by the image processing apparatus 3.

The endoscope 2 includes an image capturing unit 22 for capturing an image at a leading edge of a cylindrical casing (insertion unit) 21 while having a circuit unit (not illustrated), which converts the image captured by the image capturing unit 22 into an electric signal, accommodated in the casing 21. The electric signal obtained by the conversion in the circuit unit is output to the image processing apparatus 3 via a connection cable 5 guided out of a trailing edge of the endoscope 2.

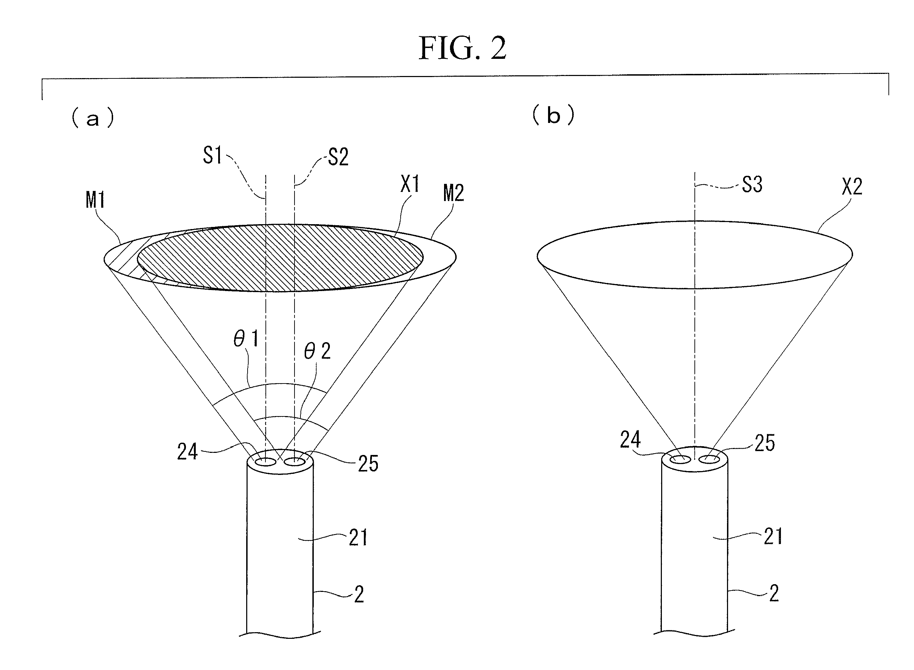

The image capturing unit 22 includes an objective optical system including a plurality of sets (two sets in the present embodiment) of photographing lenses 24 and 25. As illustrated in Fig. 2 (a), the photographing lenses 24 and 25 have their respective optical axes S1 and S2 arranged to be parallel to each other and spaced a predetermined distance apart from each other in a direction perpendicular to an axis of the casing 21 in the endoscope 2 and have a parallax therebetween. The circuit unit includes image sensors such as charge coupled devices (CCDs), respectively, for the photographing lenses 24 and 25, and outputs images respectively captured via the photographing lenses 24 and 25 as electric signals to the image processing apparatus 3.

The image processing apparatus 3 performs processing based on a computer program previously installed, to process images respectively captured via the photographing lenses 24 and 25, and generates an electric signal of a display image to be displayed by the image display apparatus 4. The image processing apparatus 3 and the image display apparatus 4 are connected to each other via a connection cable 6. The electric signal generated by the image processing apparatus 3 is output to the image display apparatus 4.

The image display apparatus 4 includes an image display unit 41, and displays the display image based on the electric signal input from the image processing apparatus 3 in the image display unit 41.

Next, a specific configuration of the stereoscopic endoscope system 1 according to the present embodiment will be described below.

In the present embodiment, respective angles of view θ1 and θ2 of the photographing lenses 24 and 25 constituting the image capturing unit 22 in the endoscope 2 are the same.

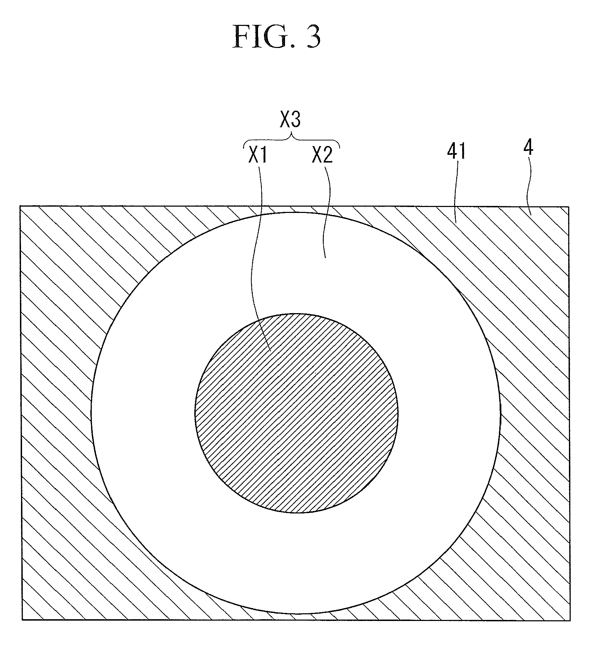

As illustrated in Figs. 2 and 3, in the image processing apparatus 3, data representing a stereoscopic image (three-dimensional image) X1 having a parallax is generated from an image M1 acquired via the photographing lens 24 and an image M2 acquired via the photographing lens 25. The stereoscopic image X1 is formed in an area where the image M1 captured via the photographing lens 24 and the image M2 captured via the photographing lens 25 overlap each other.

In the image processing apparatus 3, processing for combining the image M1 and the image M2 is performed, to generate data representing a planar image (two-dimensional image) X2 when photographing is performed via a virtual lens having a virtual optical axis S3 midway between the optical axis S1 of the photographing lens 24 and the optical axis S2 of the photographing lens 25, as illustrated in Fig. 2 (b).

In the image processing apparatus 3, data representing a display image X3 to be output to the image display apparatus 4 is generated from the generated stereoscopic image X1 and planar image X2.

As illustrated in Fig. 3, the display image X3 is obtained by arranging the stereoscopic image X1 in a circular area in its central part and arranging the planar image X2 in an annular area in its outer peripheral part. For example, the stereoscopic image X1 is arranged in a range within an angle of view of 60° centered around the optical axis S3, and the planar image X2 is arranged in a range within an angle of view of 100° centered around the optical axis S3.

An absolute value of distortion is preferably within 5 % for the stereoscopic image X1. If distortion between stereoscopic image generation areas of the photographing lenses 24 and 25 exceeds 5 %, image processing is preferably performed so that the absolute value of the distortion for the stereoscopic image X1 is preferably within 5 % in the image processing apparatus 3.

The display image X3 is displayed in the image display unit 41 in the image display apparatus 4 based on the data representing the display image X3.

According to the aforementioned configuration, the display image X3 displayed in the image display apparatus 4 has the stereoscopic image X1 arranged in its central part and the planar image X3 arranged in its outer peripheral part. Thus, an observer can observe the stereoscopic image X1 displayed in the central part of the display image X3 to perform a surgical treatment while observing the planar image X2 around the periphery thereof, as needed. Thus, the image to be displayed can be observed without being switched so that usability is improved.

At this time, the periphery of the stereoscopic image X1 is the planar image X2. Thus, the observation can be performed using a good image with no distortion.

Next, another plurality of embodiments of the present invention will be described below. In each of the embodiments, described below, an overall configuration of a stereoscopic endoscope system 1 is as illustrated in the aforementioned first embodiment. A configuration different from that in the first embodiment will mainly be described below.

### {Second Embodiment}

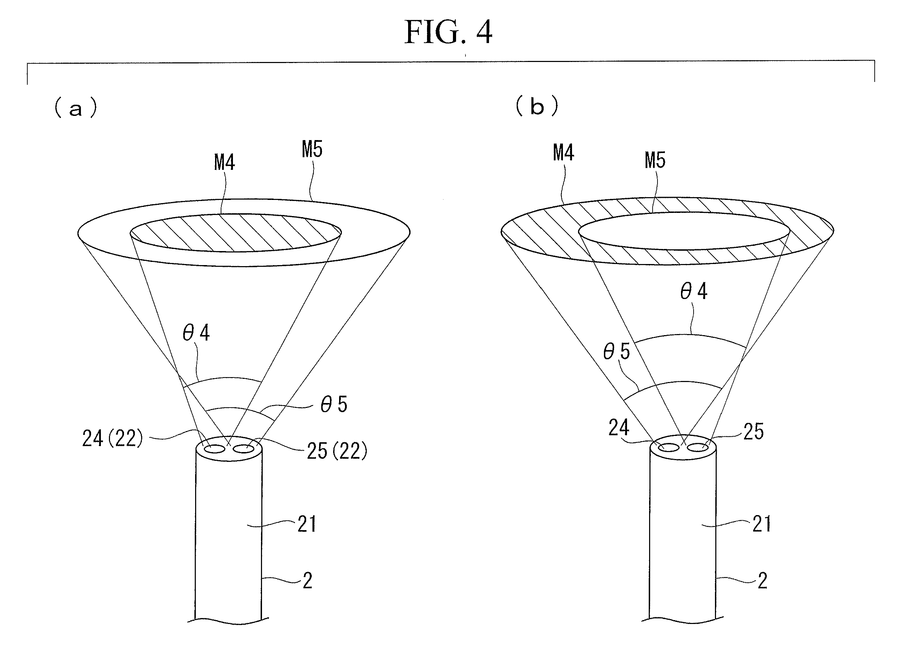

As illustrated in Fig. 4, in the present embodiment, photographing lenses 24 and 25 constituting an image capturing unit 22 in an endoscope 2 are set so that either one of their angles of view θ4 and θ5 or their diameters becomes larger depending on the dominant eye of an observer.

If the dominant eye of the observer is a right eye, as illustrated in Fig. 4 (a), for example, the angle of view θ5 of one, which is positioned on the right side of an image capturing target, of the photographing lenses 24 and 25, for example, the photographing lens (first image capturing unit) 25 is set to be larger than the angle of view θ4 of the other photographing lens (second image capturing unit) 24 (e.g., the angle of view θ4 = 60° and the angle of view θ5 = 120°). If the dominant eye of the observer is a left eye, as illustrated in Fig. 4 (b), the angle of view θ4 of the photographing lens (first image capturing unit) 24, for example, is set to be larger than the angle of view θ5 of the other photographing lens (second image capturing unit) 25.

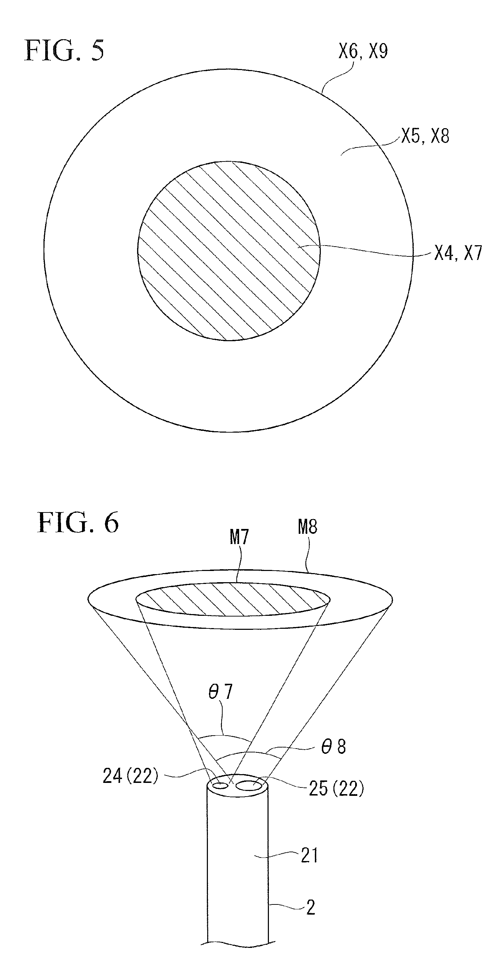

As illustrated in Figs. 4 and 5, in an image processing apparatus 3, data representing a stereoscopic image (three-dimensional image) X4 having a parallax is generated from an image M4 captured by the photographing lens 24 and an image M5 captured by the photographing lens 25. The stereoscopic image X4 is formed in an area where the image M4 captured by the photographing lens 24 and the image M5 captured by the photographing lens 25 overlap each other (an area of the images M4 and M5 having the smaller one of the respective angles of view θ4 and θ5 of the photographing lenses 24 and 25).

In the image processing apparatus 3, the image M4 or M5 having the larger one of the respective angles of view θ4 and θ5 of the photographing lenses 24 and 25 is used as a planar image (two-dimensional image) X5, to generate data.

That is, in the present embodiment, in the image processing apparatus 3, the image (first image) M5 having the larger angle of view θ5 is applied to the stereoscopic image X4 and the planar image X5, and the image (second image) M4 having the smaller angle of view θ4 is overlaid on the center thereof, to perform image processing.

In the image processing apparatus 3, data representing a display image X6 to be output to the image display apparatus 4 is generated from the generated stereoscopic image X4 and planar image X5.

The display image X6 is obtained by arranging the stereoscopic image X4 in a circular area in its central part and arranging the planar image X5 in an annular area in its outer peripheral part.

The display image X6 is displayed in the image display unit 41 in the image display apparatus 4 based on the data representing the display image X6.

Even by the aforementioned configuration, the display image X6 displayed in the image display apparatus 4 has the stereoscopic image X4 arranged in its central part and the planar image X5 arranged in its outer peripheral part. Thus, the observer can observe the stereoscopic image X4 displayed in the central part of the display image X6 to perform a surgical treatment while observing the planar image X5 around the periphery thereof, as needed. Thus, the image to be displayed can be observed without being switched so that usability is improved.

At this time, the periphery of the stereoscopic image X1 is the planar image X5. Thus, the observation can be performed using a good image with no distortion.

Further, by the configuration according to the present embodiment, the stereoscopic image X4 is obtained by combining the images M4 and M5 respectively acquired via the photographing lenses 24 and 25. However, the planar image X5 around the periphery thereof uses the images M4 and M5 acquired via the photographing lenses 24 and 25 as they are so that the image processing in the image processing apparatus 3 can be reduced.

### {Third Embodiment}

As illustrated in Fig. 6, in the present embodiment, photographing lenses 24 and 25 constituting an image capturing unit 22 in an endoscope 2 are arranged so that their respective optical axes S1 and S2 are parallel to each other and spaced a predetermined distance apart from each other in a direction perpendicular to an axis of a casing 21 in the endoscope 2. The photographing lenses 24 and 25 are set so that their respective numbers of pixels are the same and either one of their respective angles of view θ7 and θ8 becomes larger. In the present embodiment, the angle of view θ8 of the photographing lens (first image capturing unit) 25 is set larger than the angle of view θ7 of the photographing lens (second image capturing unit) 24.

As illustrated in Figs. 5 and 6, in an image processing apparatus 3, data representing a stereoscopic image (three-dimensional image) X7 having a parallax is generated from an image (second image) M7 acquired via the photographing lens 24 and an image (first image) M8 acquired via the photographing lens 25. The stereoscopic image X7 is formed in an area where the image M7 acquired via the photographing lens 24 and the image M8 acquired via the photographing lens 25 overlap each other, i.e., an area of the image M7 captured by the photographing lens 24 having the smaller angle of view θ7.

In the image processing apparatus 3, the image M8 of the photographing lens 25 having the larger angle of view θ8 is used as a planar image (two-dimensional image) X8, to generate data.

As illustrated in Fig. 6, in the image processing apparatus 3, data representing a display image X9 to be output to an image display apparatus 4 is generated from the generated stereoscopic image X7 and planar image X8.

The display image X9 is obtained by arranging the stereoscopic image X7 in a circular area in its central part and arranging the planar image X8 in an annular area in its outer peripheral part.

The display image X9 is displayed in an image display unit 41 in the image display apparatus 4 based on the data representing the display image X9.

Even by the aforementioned configuration, the display image X9 displayed in the image display apparatus 4 has the stereoscopic image X7 arranged in its central part and the planar image X8 arranged in its outer peripheral part. Thus, an observer can observe the stereoscopic image X7 displayed in the central part of the display image X9 to perform a surgical treatment while observing the planar image X8 around the periphery thereof, as needed. Thus, an image to be displayed can be observed without being switched so that usability is improved.

At this time, the periphery of the stereoscopic image X7 is the planar image X8. Thus, the observation can be performed using a good image with no distortion.

Further, by the configuration according to the present embodiment, the number of pixels, in a region of the stereoscopic image X7, of the photographing lens 24 having the smaller angle of view θ8 out of the photographing lenses 24 and 25 becomes larger than that of the other photographing lens 25. Thus, a resolution in the stereoscopic image X7 can be made higher than that in the first embodiment.

### {Other embodiments}

Configurations, as described below, can also be combined with the configurations illustrated in the first to third embodiments.

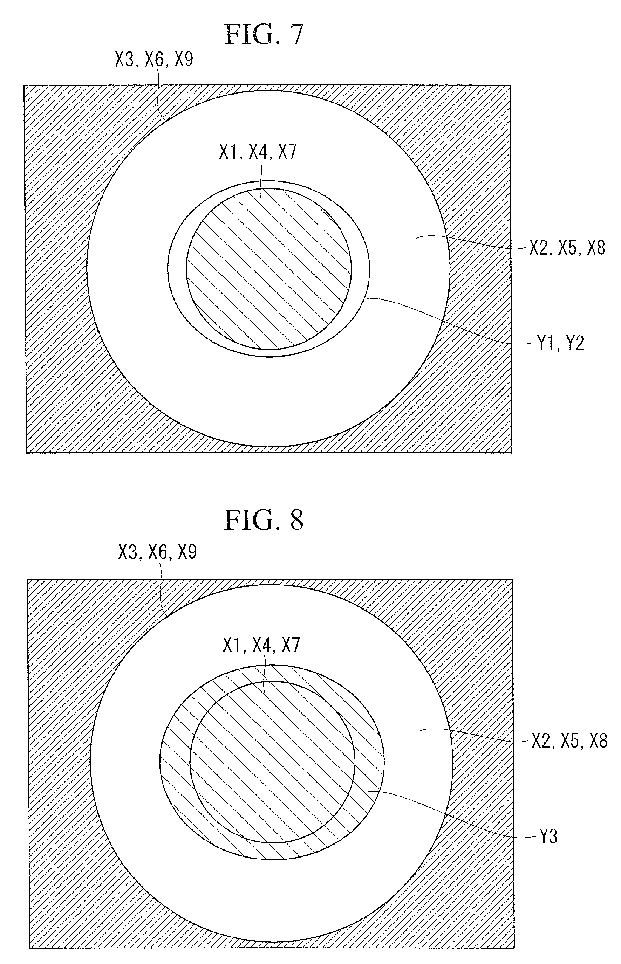

In the first to third embodiments, the display images X3, X6, and X9 displayed in the image display apparatus 4 respectively have the stereoscopic images X1, X4, and X7 arranged in their respective central parts and the planar images X2, X5, and X8 arranged in their respective outer peripheral parts, as illustrated in Fig. 7. Annular boundary regions between the stereoscopic images X1, X4, and X7 and the planar images X2, X5, and X8 in the display images X3, X6, and X9 can be used as luminance reduction portions Y1 by the image processing of the image processing apparatus 3. The luminance reduction portions Y1 have their respective luminances reduced to approximately one-third, for example, of those of the stereoscopic images X1, X4, and X7 and the planar images X2, X5, and X8.

The luminance reduction portions Y1 can respectively make annular boundary regions between the stereoscopic images X1, X4, and X7 and the planar images X2, X5, and X8 inconspicuous in the display images X3, X6, and X9. As a result, an uncomfortable feeling caused by the stereoscopic images X1, X4, and X7 and the planar images X2, X5, and X8 around the outer peripheries thereof being discontinuous can be suppressed.

As illustrated in Fig. 7, the annular boundary regions between the stereoscopic images X1, X4, and X7 and the planar images X2, X5, and X8 in the display images X3, X6, and X9 can respectively be used as line portions (boundary display portions) Y2 having a fixed width by the image processing of the image processing apparatus 3. The line portions Y2 can respectively be generated along outer peripheral edges of the stereoscopic images X1, X4, and X7, for example.

The line portions Y2 respectively make annular boundary portions between the stereoscopic images X1, X4, and X7 and the planar images X2, X5, and X8 definite in the display images X3, X6, and X9. Moreover, the line portion Y2 having a fixed width can definitely divide both sides of the boundary portion. As a result, an uncomfortable feeling caused by the stereoscopic images X1, X4, and X7 and the planar images X2, X5, and X8 around the outer peripheries thereof being discontinuous can be suppressed.

Further, as illustrated in Fig. 8, in annular boundary portions Y3 between the stereoscopic images X1, X4, and X7 and the planar images X2, X5, and X8 in the display images X3, X6, and X9, respective parallaxes between the images M1, M4, and M7 and the images M2, M5, and M8 forming the stereoscopic images X1, X4, and X7 can be generated to gradually decrease toward the outer peripheries by the image processing of the image processing apparatus 3. At this time, in outermost peripheral parts of the boundary portions Y3, the parallaxes between the images M1, M4, and M7 and the images M2, M5, and M8 are preferably the same as those in the planar images X2, X5, and X8, i.e., zero.

As a result, an uncomfortable feeling caused by the stereoscopic images X1, X4, and X7 and the planar images X2, X5, and X8 around the outer peripheries thereof being discontinuous can be suppressed.

In addition thereto, the various configurations illustrated in the aforementioned embodiments can be changed, as needed, without departing from the scope of present invention.

### {Reference Signs List}

- 1: stereoscopic endoscope system
- 2: endoscope
- 3: image processing apparatus
- 4: image display apparatus
- 5: connection cable
- 6: connection cable
- 21: casing (insertion unit)
- 22: image capturing unit
- 24: photographing lens (first image capturing unit, second image capturing unit)
- 25: photographing lens (second image capturing unit, first image capturing unit)
- 41: image display unit
- M4, M8: image (first image)
- M5, M7: image (second image)
- X1, X4, X7: stereoscopic image (three-dimensional image)
- X2, X5, X8: planar image (two-dimensional image)
- X3, X6, X9: display image
- Y1: luminance reduction portion
- Y2: line portion (boundary display portion)
- Y3: boundary portion

## Claims

1. A stereoscopic endoscope system comprising:
an endoscope including a plurality of image capturing units which photograph a subject to acquire images, at a leading edge of a long narrow insertion unit insertable into the subject;
an image processing apparatus that processes the images acquired by the endoscope to generate a display image; and
an image display apparatus that displays the display image generated by the image processing in the image processing apparatus,
wherein the image processing apparatus generates the display image having a three-dimensional image generated from the plurality of images acquired by the plurality of image capturing units arranged in a central part of the display image and having a two-dimensional image arranged around the outer periphery of the three-dimensional image.

2. The stereoscopic endoscope system according to claim 1, wherein the image processing apparatus generates the three-dimensional image from a first image acquired by a first image capturing unit and a second image acquired by a second image capturing unit and having a parallax from the first image, and obtains the two-dimensional image from the first image.

3. The stereoscopic endoscope system according to claim 2, wherein the first image capturing unit has a larger angle of view than that of the second image capturing unit.

4. The stereoscopic endoscope system according to claim 3, wherein the endoscope performs photographing by arranging the first image capturing unit having the larger angle of view than that of the second image capturing unit on the side of the dominant eye of a user.

5. The stereoscopic endoscope system according to any one of claims 2 to 4, wherein the first image capturing unit and the second image capturing unit have the same number of pixels.

6. The stereoscopic endoscope system according to claim 1, wherein the image processing apparatus generates the three-dimensional image from the first image acquired by the first image capturing unit and the second image acquired by the second image capturing unit and having a parallax from the first image, generates a third image having an optical axis midway between the first image capturing unit and the second image capturing unit from the first image and the second image, and uses the third image as the two-dimensional image.

7. The stereoscopic endoscope system according to claim 6, wherein the first image capturing unit has a larger angle of view than that of the second image capturing unit.

8. The stereoscopic endoscope system according to claim 6 or 7, wherein the first image capturing unit and the second image capturing unit have the same number of pixels.

9. The stereoscopic endoscope system according to any one of claims 1 to 8, wherein the image processing apparatus sets a luminance of a boundary portion between the three-dimensional image and the two-dimensional image lower than those of the three-dimensional image and the two-dimensional image.

10. The stereoscopic endoscope system according to any one of claims 1 to 8, wherein the image processing apparatus generates a boundary display portion representing a boundary between the three-dimensional image and the two-dimensional image.

11. The stereoscopic endoscope system according to any one of claims 1 to 8, wherein the image processing apparatus generates the three-dimensional image from the first image acquired by the first image capturing unit and the second image acquired by the second image capturing unit and having a parallax from the first image while gradually reducing the parallax between the first image and the second image, which form the three-dimensional image, toward the two-dimensional image from the three-dimensional image in the boundary portion between the three-dimensional image and the two-dimensional image.
